Europäisches Patentamt

(19) European Patent Office

Office européen des brevets

(11) Numéro de publication: **0 125 349**

**A2**

(12) **DEMANDE DE BREVET EUROPEEN**

(21) Numéro de dépôt: 83201399.9

(22) Date de dépôt: 30.09.83

(30) Priorité: 20.04.83 IT 6742883
29.06.83 IT 6771483

(43) Date de publication de la demande:
21.11.84 Bulletin 84/47

(84) Etats contractants désignés:
AT BE CH DE FR GB LI LU NL SE

(51) Int. Cl.³: **H 05 G 1/44**
**G 03 B 41/16, A 61 B 6/14**

(71) Demandeur: **X-RAY HOLDING S.A.**
**Boulevard Royal 2**
**Luxembourg(LU)**

(72) Inventeur: **Pasquarelli, Aldo**
**Via Marco Polo 40**
**I-10129 Torino(IT)**

(74) Mandataire: **Patrito, Pier Franco, Dr. Ing.**
**Cabinet PATRITO BREVETTI Via Don Minzoni 14**
**I-10121 Torino(IT)**

(54) Dosimètre pour emploi en radiographie et spécialement pour la radiographie endo-orale.

㊗ Un dosimètre pour radiographie endoorale comprenant un capteur blindé (S) à introduire dans la bouche du patient avec un film radiographique (P) et contenant un écran de conversion X-optique (3) qui excite des éléments photosensibles (2) connectés, au moyen d'un câble blindé (C), à un circuit élaborateur (B) pour compenser les défauts de fonctionnement dérivant de la possible baisse de la tension d'alimentation (A) et commander un circuit d'interruption (K), branché en série avec l'appareil radiographique (4), quand le film (P) a reçu la dose d'irradiation considérée optimale. Le circuit d'interruption (K) est associé à un circuit de sécurité (Z) et peut être branché entre la prise d'alimentation (A) et la fiche du câble d'alimentation (L) de L'appareil radiographique (4).

FIG. 1

# DOSIMETRE POUR EMPLOI EN RADIOGRAPHIE ET
## SPECIALEMENT POUR LA RADIOGRAPHIE ENDOORALE

La présente invention a pour objet un dosimètre pour emploi radiographique, spécialement mais non exclusivement pour la radiographie endoorale, comprenant un écran de conversion X-optique, destiné à être disposé derrière un film radiographique, du côté opposé à un générateur de rayons X par rapport au sujet à radiographier, des éléments photosensibles disposés pour être excités par la lumière émise par ledit écran de conversion quand il est frappé par les rayons X, un circuit intégrateur, disposé pour intégrer le signal électrique produit par lesdites éléments photosensibles, et un circuit d'interruption inséré dans le circuit d'alimentation du générateur de rayons X pour en interrompre le fonctionnement lors que le signal de sortie dudit circuit intégrateur arrive à une valeur préfixée.

Les dosimètres pour emploi radiographique ont le but de mesurer directement l'irradiation reçue par le film radiographique pendant la prise de vue, dans le but d'arrêter le fonctionnement du générateur de rayons X dès que la dose opportune d'irradiation a été atteinte, pour éviter ainsi que l'opérateur doive calculer, en fonction des conditions opératives, le temps d'exposition nécessaire et doive ajuster sur ce temps le temporisateur généralement inclus dans l'appareil radiographique. Des types différents de dosimètres sont connus pour l'emploi en radiographie générale, mais des difficultés ont été rencontrées pour réaliser des dosimètres appropriés pour des emplois spécifiques, tels que, en particulier, la radiographie endoorale. Suivant la demande de Brevet allemande No. 24 43 681 il est connu un dosimètre pour la radiographie endoorale, mais il est ainsi fait qu'il exige que l'on introduit dans la bouche du patient l'extrémité du générateur de rayons X, ce qui est indésirable et, de plus, empêche certains types de prises de vue. Par le Brevet français No. 76 12 408 on connait un dosimètre pour la radiographie endoorale qui n'a pas un tel défaut, mais suivant lequel on doit introduire dans la bouche du patient un révélateur pourvu d'un émetteur d'ondes électromagnétiques, lesquelles sont reçues par un récepteur externe. Ce système est peu fiable en vue des troubles qui peuvent dériver de la présence, dans la bouche du patient, de prothéses on de squelettes métalliques, ainsi que de la présence dans l'endroit d'inductions électromagnétiques, désormais diffusées partout à des niveaux relativement élevés.

L'emploi de dosimètres en radiographie endoorale, pratiquée en général par des dentistes qui branchent l'appareil radiographique sur un réseau de distribution sujet à des baisses de tension, présente en outre des problèmes particuliers parce qu'une baisse considérable de la tension du réseau produit, en plus de la réduction de l'intensité d'émission du générateur de rayons X, une modification du spectre de fréquences émis, avec la conséquence d'une différente variation de l'absorption entre le métal, par lequel certains types de films radiographiques sont blindés, et d'autres matériaux. Comme conséquence, la relation entre l'irradiation efficace reçue par le film et celle qui est reçue par l'écran de conversion X-optique se modifie. En outre le déplacement vers des conditions de faible irradiation modifie le comportement de l'écran de conversion X-optique, des éléments photosensibles et d'autres composants des circuits, de sorte que, dans l'ensemble, le dosimètre perd sa sensibilité et cesse d'être fiable.

En outre il serait à propos qu'un dosimètre pour radiographie endoorale pût être rendu indépendant de l'appareil radiographique, dans le sens qu'il pût lui être connecté simplement en le branchant en série entre la fiche de l'appareil radiographique et la prise d'alimentation, sans porter aucune modification à l'appareil auquel le dosimètre est annexé. Cela suppose entre autre que, après un cycle de fonctionnement, les circuits du dosimètre puissent être rétablis sans comporter des pertes de temps, et préférablement d'une façon automatique.

Enfin, en cas de dommage à la commande de mise en fonction avec dispositif "à homme mort", dont les appareils de radiographie sont généralement pourvus, ceux-ci peuvent entrer en fonction de façon intempestive et éventuellement inaperçue, lors de leur connexion au réseau, et s'ils sont pourvus de dosimètre on peut aussi constater une répétition périodique non voulue de la mise en fonction, due à la remise en fonction automatique du dosimètre. Il est donc à propos que le dosimètre appliqué à l'appareil radiographique soit en condition d'introduire une sûreté contre des cas semblables.

Le premier but de l'invention est donc de réaliser un dosimètre pour radiographie qui, en surmontant les inconvénients des dosimètres précédemment connus, soit aussi et particulièrement approprié pour des usages spécifiques, et spécialement pour la radiographie endoorale.

Ce but est atteint, suivant l'invention, dans un dosimètre du type indiqué dans la prémise, par le fait qu'un support pour le film radiographique, l'écran de conversion X-opique et les éléments photosensibles forment un organe capteur de dimensions appropriées pour en permettre l'introduction dans la cavité orale; le circuit intégrateur est constructivement séparé dudit organe capteur et il est connecté auxdits éléments photosensibles par un câble; et le dispositif comprend des moyens circuitaux pourvus de moyens de réglage manuel et/ou automatique pour conformer les caractéristiques du dosimètre aux différentes conditions opératives.

Grâce à ces caractéristiques, l'organe capteur du dosimètre peut être introduit dans la bouche du patient tandis que l'appareil radiographique demeure à l'extérieur; l'organe capteur introduit dans la bouche ne demande pas de comprendre un émetteur d'ondes électromagnétiques, ni d'autres circuits, et il peut donc resulter petit et léger; la transmission du signal de l'organe capteur au circuit intégrateur extérieur a lieu à travers un câble et donc avec une sensibilité réduite aux troubles venant des inductions électromagnétiques et sans aucune sensibilité à la présence éventuelle de prothèses ou de sque-

- 4 -

0125349

lettes métalliques dans la bouche du patient; et la réponse du dosi-mètre peut être conformée exactement, de façon manuelle ou automatique, aux différentes conditions opératives, et particulièrement à l'usage de films blindés ou non blindés et aux variations de la tension d'alimentation.

De préférence ledit organe capteur est étanche à la lumière et il est blindé par rapport aux inductions électromagnétiques, et le câble qui relie les éléments photosensibles au circuit intégrateur est lui aussi blindé. On évite ainsi tout trouble au fonctionnement du dosimètre, même en des locaux où il y a des inductions électromagnétiques très fortes.

De préférence lesdits moyens circuitaux du dosimètre comprennent un comparateur de seuil ayant une tension de seuil variable, dans lequel le signal du circuit intégrateur est confronté avec ladite tension de seuil pour produire un signal de sortie destiné à comman-der ledit circuit d'interruption. Il est préféré que ledit compara-teur comprende un générateur de tension de seuil qui se modifie, en fonction de la tension d'alimentation du générateur de rayons X, de façon telle qu'elle tende à compenser les variations de la réponse globale du système aux variations de ladite tension d'alimentation. Cette variation de la tension de seuil par rapport à la tension d'alimentation peut être linéaire ou non linéaire. De cette façon on peut obtenir une compensation automatique des effets de la variation de la tension d'alimentation, même dans un champ très étendu.

De préférence ledit circuit intégrateur comprend au moins deux condensateurs qu'on peut commuter pour modifier la constante d'intégration du circuit. Cela, éventuellement en combinaison avec un réglage manuel de la tension de seuil du comparateur, permet de conformer la réponse du dosimètre spécialement à l'emploi de films de types différents. La commutation des condensateurs peut aussi être automatique, et être commandée par le niveau du signal produit par les éléments photosensibles.

Il est aussi un but de l'invention celui de réaliser un dosimètre pour radiographie qui puisse être appliqué à un appareil radiographique simplement en le branchant entre la fiche et la prise d'alimentation de l'appareil radiographique.

Ce but est atteint, suivant l'invention, par le fait que ledit circuit d'interruption comprend des moyens pour le retenir dans la condition d'interruption, qui sont actifs pour un temps supérieur à la période d'exposition maximum permise par l'appareil radiographique auquel est destiné le dosimètre. De plus, ledit circuit intégrateur comprend préférablement des moyens de mise au zéro graduelle et spontanée qui agissent dans un temps inférieur à la période d'activité desdits moyens de retenue du circuit interrupteur. Grâce à ces caractéristiques, on évite que l'appareil radiographique répète intempestivement une prise de vue après l'interruption produite par le dosimètre, sans demander que les circuits du dosimètre soient connectés aux circuits intérieurs de l'appareil radiographique; et de plus, soit l'appareil radiographique que le dosimètre se présentent spontanément prêts pour une nouvelle exposition dès que la période d'activité des moyens de retenue du circuit interrpteur est terminée.

Enfin, il est encore un but de l'invention celui de réaliser un dosimètre pour radiographie qui soit capable d'empêcher l'exécution de prises de vue non commandées, même en cas d'endommagement des moyens de sûreté de l'appareil radiographique, ou d'erreurs de manoeuvre.

Ce but est atteint, suivant l'invention, per le fait que le dosimètre comprend un moyen interrupteur.sensible à l'intensité de courant, destiné à être connecté en série au circuit d'alimentation de l'appareil radiographique et prédisposé pour interrompre ledit circuit au dépassement d'une valeur établie à l'avance du courant absorbé par l'appareil, valeur comprise entre l'absorption de l'appareil en conditions d'attente et son absorption en conditions d'activité; un relais dont l'enroulement est relié en parallèle au circuit d'alimen-

tation en aval dudit moyen interrupteur sensible à l'intensité de courant et dont le contact est disposé pour court-circuiter ledit moyen interrupteur sensible à l'intensité de courant; et un moyen de retard associé audit relais et prédisposé pour en différer l'intervention pour un temps supérieur au temps d'intervention dudit moyen interrupteur sensible à l'intensité de courant.

Grâce a cette caractéristique, lors que les moyens de remise en fonction du dosimètre rétablissent l'alimentation à l'appareil radiographique après une interruption, le dispositif examine immédiatement si l'absorption de courant de l'appareil radiographique correspond (comme elle devrait) à une condition d'attente, et dans ce cas le moyen interrupteur sensible à l'intensité de courant n'intervient pas et, après le témps établi par les moyens de retard, il est court-circuité par le relais, après quoi l'appareil radiographique peut être librement mis en fonction. Si, au contraire, suite à tous genres d'anomalies, l'appareil radiographique absorbe un courant tel à montrer qu'il va entrer en fonction, le moyen sensible à l'intensité de courant interrompe immédiatement le circuit d'alimentation en empêchant ainsi le fonctionnement intempestif de l'appareil radiographique.

Lesdites caractéristiques et d'autres, et les avantages de l'objet de l'invention, apparaîtront plus clairement de la suivante description de certaines formes de rélisation données à titre d'exemples et non limitatives, représentées schématiquement dans les dessins annexées, dans lesquels :

la figure 1 est un schéma général de la disposition du dosimètre en relation à l'appareil radiographique qu'il contrôle;

la figure 2 est un schéma électrique partiellement à blocs des circuits du dosimètre;

la figure 3 montre avec plus de détails un schéma de circuit pour la génération d'une tension de référence variable de manière linéaire;

la figure 4 montre, de façon analogue à la figure 3, un schéma de circuit approprié pour élaborer une tension de référence variable de manière linéaire, pour produire une tension de référence variable non linéairement;

les figures 5 et 6 sont des diagrammes relatifs aux tensions générées par les circuits, respectivement, selon les figures 3 et 4;

la figure 7 est une vue frontale du capteur, et

la figure 8 montre, à plus grande échelle, une section partielle du capteur, faite selon la ligne VIII-VIII de la figure 7;

la figure 9 montre le schéma d'un dispositif de sécurité faisant usage d'un fusible; et

la figure 10 montre analoguement le schéma d'un dispositif faisant usage d'un interrupteur à relais de courant maximum.

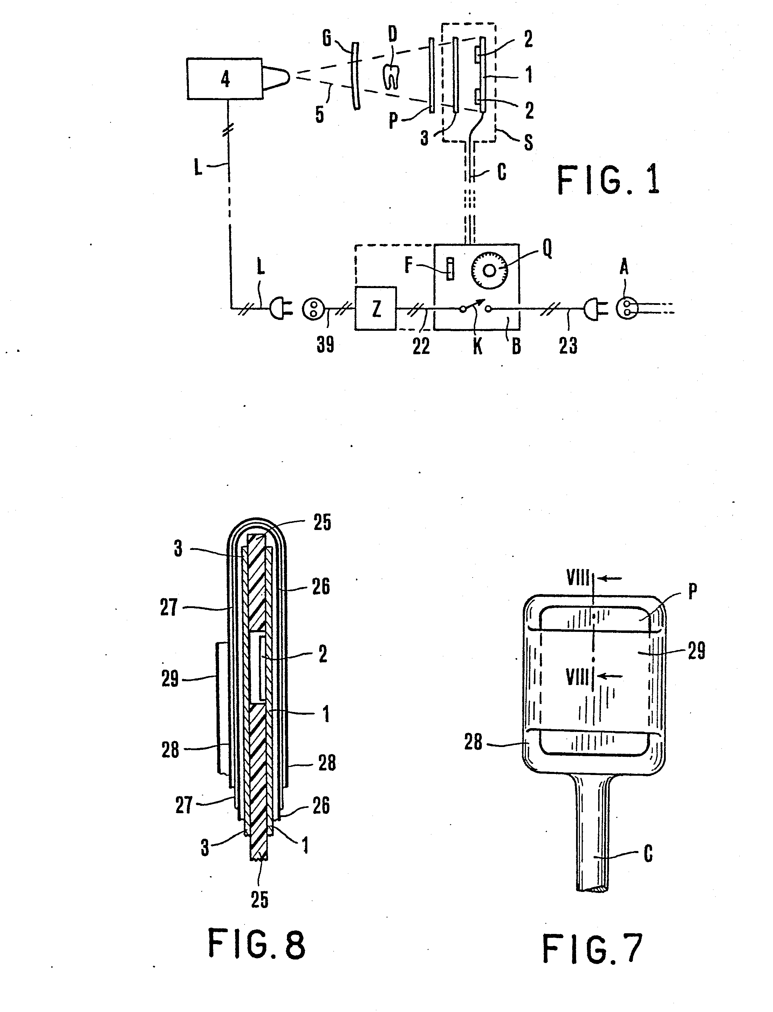

Dans la figure 1 sont indiqués sommairement le générateur 4 de rayons X d'un appareil radiographique alimenté par un câble L, un film radiographique P disposé sur le parcours 5 du faisceau de rayons X générés et une dent D, supposée comme l'objet de la radiographie à effectuer; G indique une partie de la joue ou lèvre du patient; le film P est disposé dans la cavité orale tandis que le générateur 4 de rayons X est approché à la joue ou lèvre 6 de l'extérieur. C'est donc la disposition qu'il faut adopter pour des radiographies dentaires endoorales et similaires.

Derrière le film P est disposé un écran de conversion X-optique 3 observé par des éléments photosensibles 2 appliqués sur une plaque de support 1. Les éléments photosensibles 2 sont reliés par un câble C à un dispositif d'élaboration de signal B, comprenant un interrupteur K inséré sur la ligne L d'alimentation (qui arrive d'une prise A) de l'appareil radiographique 4. Le circuit B est disposé pour ouvrir l'interrupteur K (normalement fermé) lors que le film P a reçu la dose d'irradiation appropriée pour la meilleure réalisation de la radiographie dans les conditions opératives prévues et réalisées. Cette circonstance est verifiée au moyen de l'écran de conversion 3 qui transforme en radiation lumineuse une partie de l'

énergie des rayons X qui, en passant à travers le film, tombent sur l'écran 3, au moyen des éléments photosensibles 2 qui transforment une partie de cette radiation lumineuse en un signal électrique, et au moyen du circuit B qui élabore ce signal électrique comme indiqué plus avant. Au circuit d'élaboration B est annexé en circuit de sécurité Z.

En outre, on observe sur la figure 1 que soit l'ensemble de l'écran de conversion 3 et des éléments photosensibles 2 que le câble C sont pourvus d'un blindage S capable d'isoler lesdites parties par rapport aux inductions électromagnétiques; grâce à ce blindage, aucune trouble au fonctionnement de l'appareil n'est à craindre, même en présence de pollution électromagnétique du local de travail. On observe encore, sur la figure 1, que le dispositif B (qui dans ce cas comprend le circuit de sécurité Z) est muni de deux tronçons de câble électrique 23,39 pourvus de connecteurs respectivement femelle et mâle, appropriés pour se raccorder respectivement avec la fiche normale du câble L d'alimentation du générateur 4 de rayons X, et avec la prise de courant normale A. De cette façon, le dosimètre est relié à l'appareil radiographique dans la manière la plus simple et, grâce aux caractéristiques exposées plus avant, sans demander aucune modification ou adaptation de l'appareil.

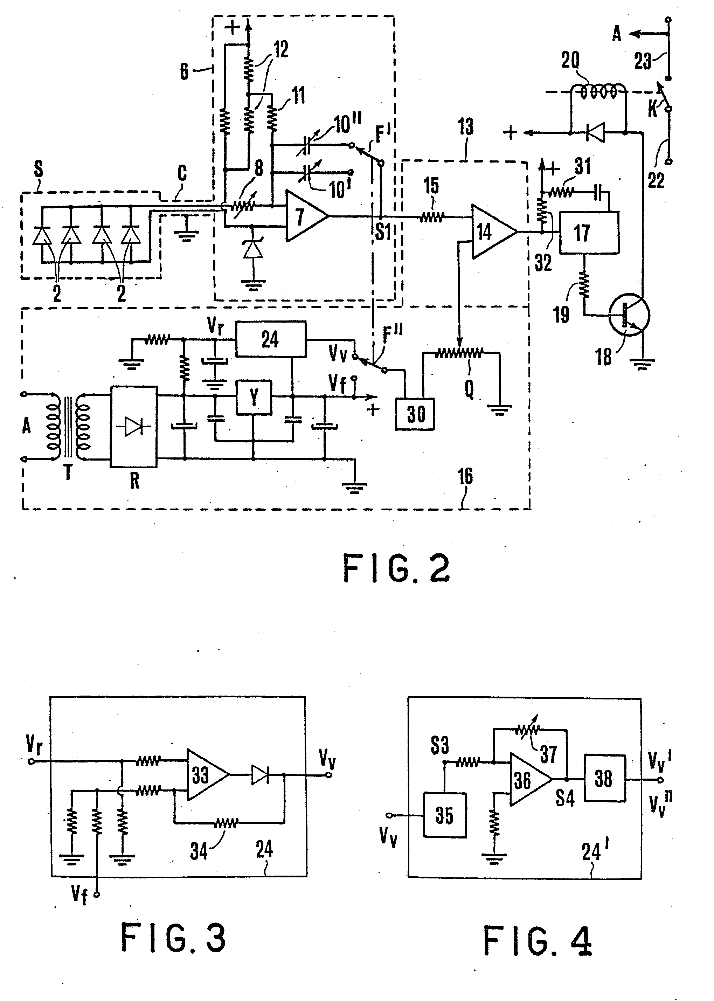

Tel qu'on le voit sur le schéma de la figure 2, les éléments photosensibles 2 (dans ce cas, des photodiodes) sont reliés, à travers le câble blindé C et à travers une résistance éventuellement réglable 8, à l'entrée d'un amplificateur opérationnel 7 qui fait partie d'un circuit intégrateur généralement indiqué par 6. Le réseau d'intégration de l'amplificateur opérationnel 7 comprend des condensateurs 10', 10" éventuellement variables, qu'on peut commuter. La variabilité de la résistance 8 et/ou des condensateurs 10, normalement, n'est pas laissée à la disposition de l'usager, et elle sert pour la mise au point du circuit intégrateur 6 pour en conformer le comportement, lors que cela est nécessaire, aux caractéristiques de l'ap-

pareil radiographique auquel le dosimètre est destiné. La commutation entre les différents condensateurs 10, qui peut être automatique ou bien être laissée au contrôle de l'usager moyennant un commutateur F', sert pour conformer le comportement de l'intégrateur à des conditions opératives différentes, particulièrement à l'emploi de films radiographiques blindés ou non blindés, à la disposition du générateur de rayons X à des distances différentes du sujet, et ainsi de suite. Un rétablissement graduel spontané de l'intégrateur est obtenu au moyen d'un faible courant inverse d'entrée, produite moyennant un partiteur de tension 11, 12. Comme on peut le comprendre, le signal de sortie de l'intégrateur 6 (présent sur la connexion S1) est une fonction de la dose d'irradiation reçue par le film P, à partir du commencement de l'exposition jusqu'à l'instant considéré.

Le signal présent en S1 est appliqué à un circuit comparateur 13, et précisément il est envoyé à travers une résistance 15 à un amplificateur opérationnel 14 en fonction de comparateur, dont l'autre entrée est reliée à un potentiomètre Q qui est mis à disposition de l'operateur et sert pour adapter, chaque fois, le dosimètre au type de film radiographique employé. Le potentiomètre Q fait partie d'un circuit générateur de tension de référence 16 et il est alimenté, à travers un variateur 30 (qui peut être lui aussi un potentiomètre) par un commutateur F" à deux positions, qui peut être commandé par l'opérateur pour choisir éventuellement entre une tension de référence fixe et une tension de référence variable en fonction de la tension d'alimentation de l'appareil. Dans ce but le commutateur F" peut relier le potentiomètre Q à un générateur de tension Vf fixe ou bien à un générateur de tension Vv variable.

Pour la génération de ces tensions, la tension d'alimentation A est transformée par un transformateur T, rectifiée en R, stabilisée en Y et nivelée, en réalisant ainsi la tension Vf qui a donc une valeur inchangeable malgré les fluctuations (dans certaines limites) de la tension d'alimentation A. En outre, la tension rectifiée en

R est aussi envoyée à un convertisseur de fonction 24 disposé pour donner à sa sortie una tension continue Vv qui est variable de manière préfixée avec la variation de la tension d'alimentation A (et donc de la tension rectifiée en R) comme il sera rendu clair plus avant. En concomitance avec la susdite commutation, il est souvent à propos qu'on modifie aussi la constante de temps de l'intégrateur, 6, et dans ce but le commutateur F" des tensions de référence et le commutateur F' des condensateurs 10', 10" de l'intégrateur peuvent avoir une commande commune F, ou bien être constitués par deux sections d'un seul commutateur, comme indiqué dans la figure 2.

L'amplificateur opérationnel comparateur 14 est prédisposé pour fournir à sa sortie un signal différent selon que le signal intégré présent en S1 est inférieur ou supérieur à la tension de référence réglée au moyen du potentiomètre Q. Ce signal de sortie est appliqué à un circuit monostable 17 prédisposé pour changer son état lorsque le signal d'entrée correspond au dépassement de la tension de référence par le signal intégré S1. Le circuit monostable 17 commande à travers un transistor 18 un relais 20, dont le contact K forme l'interrupteur disposé entre les lignes 39 et 23 destinées à être branchées en série au câble d'alimentation L de l'appareil radiographique. Le circuit monostable 17 comprend un temporisateur qui le maintient à l'état modifié pour un temps préfixé après son excitation, temps qui est déterminé par le condensateur 31 et par les résistances 32 et 33.

Selon une caractéristique de l'invention, la constante de temps du circuit monostable 17 est choisie non inférieure à la période d' exposition maximum permise par le temporisateur de l'appareil radiographique auquel le dosimètre est destiné. Grâce à cette caractéristique, toute double exposition est empêchée même dans le cas où, par inadvertance ou par accident, la commande de mise en marche de l'appareil radiographique ait été tenue en activité après la fin de l' exposition ou qu'elle soit réactivée trop tôt. En présence de cette

0125349

mesure de sécurité présentée par le dosimètre, le temporisateur de l'appareil radiographique peut être laissé toujours au maximum quand on se sert du dosimètre, en évitant ainsi toute possible interférence inopportune entre les deux dispositifs d'interruption, tout sans demander aucune modification de parties de l'appareil radiographique.

De son côté, le partiteur de tension 12,11, qui fournit à l'intégrateur 6 un faible courant de décharge du condensateur 10 pour rétablir l'intégrateur après chaque fonctionnement, est dimensionné de sorte que la période de décharge spontanée du condensateur 10 résulte inférieure, préférablement de peu, de la constante de temps du circuit monostable 17. De cette façon, lors que l'interruption opérée par le contact K cesse et l'appareil radiographique peut exécuter une nouvelle prise de vue, l'integrateur 6 lui aussi se trouve certainement déjà en condition d'éxécuter un nouveau cycle de travail.

En pratique, la constante de temps du circuit monostable 17 peut être choisie égale ou supérieure à 5 seconds, ou autre période maximum pour laquelle peut être prédisposé le temporisateur d'un appareil radiographique en considération des mesures de sécurité obligatoires ou conseillées.

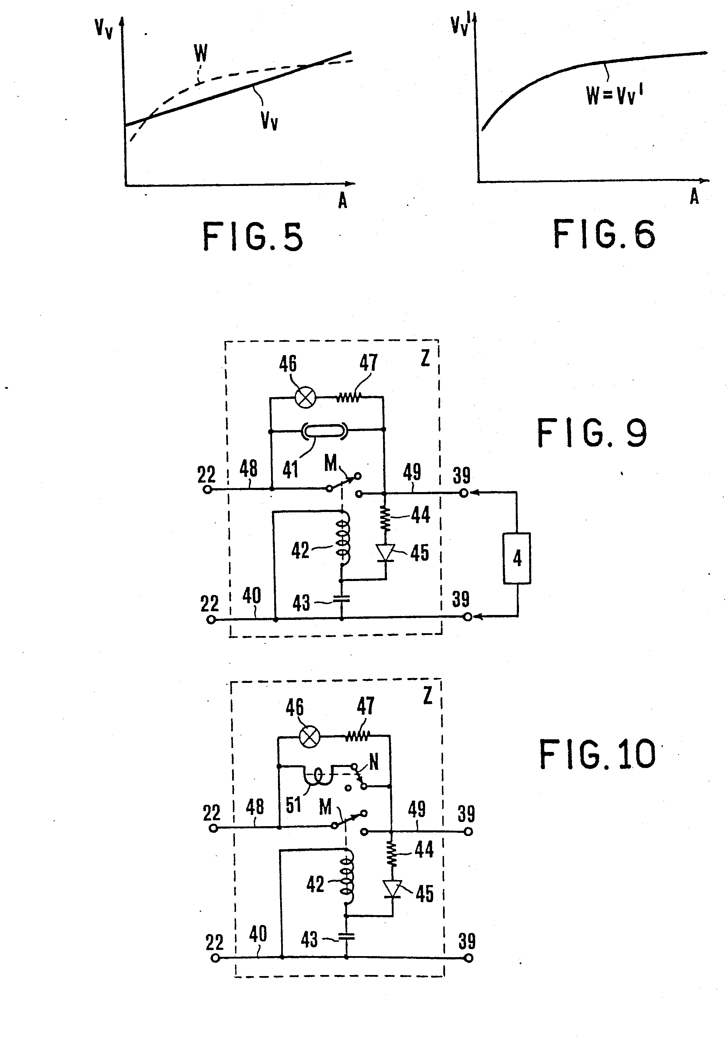

Mais les dispositions de sûreté décrites présupposent un fonctionnement correct des dispositifs de sécurité propres de l'appareil radiographique, à savoir, en général, le temporisateur qui limite la durée maximum d'exposition admise et le dispositif à homme mort appliqué à la commande de mise en fonction. Si ces deux dispositifs de sécurité sont tous les deux endommagés, la remise spontanée en fonction du dosimètre après une exposition provoque une nouvelle exposition; de même une exposition non voulue s'avère lors du branchement de l'appareil radiographique au réseau d'alimentation. Pour prévenir de telles éventualités on a prévu le dispositif de sécurité indiqué en générale par Z, dont deux formes de réalisation sont illustrées par les figures 9 et 10.

Avec référence à la figure 1, on voit que le dispositif Z est

- 12 -

0125349

branché en série entre le contact K du dosimètre et l'appareil radiographique 4. Avec référence à la figure 9, on voit que dans le dispositif de sécurité Z l'un des points de sortie 39 est directement relié à la correspondante entrée 22 par un conducteur 40, tandis que l'autre point 39 est relié à l'entrée 22 par des conducteurs 49, 48, entre lesquels est inséré le contact M normalement ouvert d'un relais 42. Les conducteurs 49, 48 sont aussi reliés entre eux par un fusible 41 dont le courant d'interruption est choisi de sorte à être compris entre le courant qui peut être absorbé par l'appareil radiographique 4 quand il est en condition d'attente, et le courant qu'il absorbe quand il commence son activité. Le temps d'intervention du fusible 41 (qui peut être un fusible rapide) est choisi de manière qu'il soit inférieur au temps nécessaire à l'appareil radiographique 4 pour commencer l'émission de rayons X.

L'enroulement du relais 42 est relié entre les conducteurs 49 et 40 en série avec une résistance 44 et une diode 45, tandis qu ' en parallèle à l'enroulement 42 est relié un condensateur 43. Une lampe témoin 46 avec résistance de limitation 47, ou autre dispositif de signalisation optique et/ou acoustique, peut être inserée en dérivation au fusible pour en signaler l'interruption.

Comme on le comprend, si lors de la clôture du contact K, ou bien lors du branchement sur le réseau d'alimentation, c'est-à-dire lors de l'application d'une tension aux points 22, l'appareil radiographique 4 est en attente et il absorbe peu de courant, le fusible 41 est capable de fournir ce courant; la resistance 44 charge le condensateur 43 tant que l'enroulement 42, suffisamment excité, ferme le contact M en mettant en court-circuit le fusible 41 et en permettant ensuite d'activer l'appareil radiographique 4. Si, au contraire, pour toute raison, ce dernier est prédisposé pour le fonctionnement et il absorbe dès le début un fort courant, le fusible 41 fond et le circuit reste interrompu, ainsi prévenant l'activation de l'appareil radiographique 4. La lampe témoin 46 signale ce qui est arrivé. La remise en fonction

- 13 -                                    0125349

du circuit exige la substitution du fusible 41.

Le circuit selon la figure 10 est identique à celui de la figure 9, sauf que pour la substitution du fusible 41 par un interrupteur 51 à relais de courant maximum ayant son contact N normalement fermé. Le dimensionnement du relais 51 suit les mêmes critères exposés pour le fusible 41. Le fonctionnement du circuit est identique à celui qu'on a exposé pour le circuit de la figure 9, excepté en ce que la remise en fonction en cas d'interruption ne demande que la manoeuvre manuelle de remise en fonction de l'interrupteur N de courant maximum.

L'emploi d'une tension de référence Vv variable, au lieu d'une tension de référence fixe Vf, est d'importance particulière quand on travaille avec des films radiographiques blindés en plomb ou autre metal, qui d'un côté réduit fortement l'intensité de la radiation qui arrive à l'écran de conversion 3, et d'autre part absorbe les radiations X de manière différente selon leur spectre de fréquence et, par conséquent, selon la tension d'alimentation du générateur 4, en provoquant des fortes variations de la réponse globale du système dosimétrique.

Una correction généralement suffisante est obtenue par une variation linéaire de la tension de référence Vv en fonction de la tension d'alimentation A, capable d'interpoler linéairement une courbe W représentant la réponse complexive du système, comme schématisé par la figure 5. Une telle tension Vv peut être obtenue, par exemple, avec un circuit 24 comme le circuit selon la figure 3, dans lequel l'amplificateur operationnel 33 amplifie, avec un gain constant défini par la résistance 34, la différence entre la tension Vr (transformée et rectifiée à partir de la tension d'alimentation A) et la tension stabilisée Vf (laquelle, outre que pouvoir être utilisée comme tension de référence, sert pour l'alimentation des circuits du dosimètre).

Dans les cas où l'on veut obtenir una correction extrèmement précise, éventuellement en faisant coïncider du tout les courbes de

La tension de référence (dans ce cas nommée tension Vv') et de la réponse globale du système, comme le montre le diagramme de la figure 6, on peut faire recours à une élaboration ultérieure de la tension variable Vv obtenue comme indiqué plus haut, ce qui peut être fait, par exemple, avec le circuit 24' indiqué sur la figure 4. Dans ce cas la tension Vv, proportionnelle à la variation de la tension d'alimentation A par rapport à une valeur de référence fixe, est appliquée à un circuit 35 qui en détermine le logarithme, de sorte qu'au point S3 est présente une tension $\ln Vv$. Cette tension est amplifiée par l'amplificateur opérationnel 36 avec un gain n déterminé par la résistance 37 (qui dans ce cas est variable pour des buts de mise à point) de sorte qu'au point S4 est présente une tension $n \ln Vv$. Enfin, cette tension est appliquée à un circuit 38 qui en détermine l'antilogarithme, en parvenant ainsi à une tension $Vv' = Vv^{n}$ dont la courbe, par un choix opportun des paramètres, peut être approximée, jusqu' éventuellement à l'identification, à la courbe W de la réponse d'ensemble du système.

Pour la réalisation pratique d'un capteur comprenant l'écran de conversion 3 et les éléments sensibles à la lumière 2, il peut être avantageux d'adopter une construction selon la figure 5. Les éléments photosensibles 2 sont réalisés ou appliqués sur un circuit imprimé 1 sur lequel est appuyée une pièce d'espacement 25 en matériau approprié, par exemple en cuir synthétique, présentant des fenêtres en correspondance des éléments photosensibles 2. Cette pièce d'espacement sert pour éviter une friction nuisible entre les éléments photosensibles et l'écran de conversion; en outre il a la fonction de conférer au capteur des caractéristiques de semirigidité pour le meilleur confort du patient. L'écran de conversion X-optique 3 est à son tour appuyé sur l'élément 25 et l'ensemble est enveloppé par une couche isolante 26 et renfermée par une mince feuille d'aluminium 27 qui forme le blindage S pour les inductions électromagnétiques. Tout cela est inclus dans un étui 28, par exemple en matière plastique.

Au revêtement d'aluminium 27 est relié le guipage métallique du câble
C dans lequel passent les conducteurs reliés au circuit imprimé
qui porte les éléments photosensibles 2.

L'étui 28 du capteur est avantageusement muni de moyens pour supporter, sur sa face antérieure, un film radiographique; ces moyens
peuvent être constitués par une poche, une bande, un guide ou une
corniche présentée par l'étui en matiére plastique et dans laquelle
peut être inséré un film normal pour radiographie muni de son revêtement étanche à la lumière. Dans ce cas, dans la bouche du patient
doit être introduit le capteur seulement, qui à son tour porte le
film radiographique, et on n'a pas de problèmes pour le positionnement correct du capteur par rapport au film. L'ensemble résulte de
dimensions très réduites de sorte que son introduction dans la bouche
du patient ne présente aucun problème.

Le potentiomètre Q et le commutateur F sont placés de préférence
sur un panneau frontal du dispositif élaborateur B pour permettre
à l'opérateur d'adapter le fonctionnement du dosimètre aux caractéristiques des films employés. En outre, le potentiomètre Q peut être
utilisé pour satisfaire des exigences particulières, comme celles
d'obtenir des radiographies de densité plus réduite ou plus grande,
ou bien d'obtenir des radiographies de densité normale tout en procédant par un développement abrégé. Le bouton de commande du potentiomètre Q peut présenter une graduation qui doit être mise en relation,
au moyen d'un tableau, avec les différents types de film et les différents résultats à obtenir.

Le nombre d'éléments photosensibles contenus dans le capteur peut
être de un ou plus, par exemple de quatre, préférablement avec une
disposition symétrique de manière à assurer un relevé correct de l'
irradiation reçue en des points significatifs, indépendemment du fait
que le capteur soit disposé verticalement (comme pour les radiographies des dents incisives) ou horizontalement (comme pour les radiographies des molaires). En outre, le nombre des éléments photosensi-

bles peut être augmenté, dans les limites du possible, afin de disposer du signal électrique maximum que l'on peut obtenir; dans le même but on se sert préférablement d'éléments de la plus grande efficacité dont on peut disposer. L'écran de conversion 3 peut être d'une seule pièce ou en différentes parties séparées disposées seulement en correspondance des fenêtres de l'élément intermédiaire 25. Le dispositif de remise au zéro graduelle de l'intégrateur peut être remplacé par une disposition de remise au zéro à la fin de chaque exposition.

Comme exposé plus haut, le dosimètre suivant l'invention est approprié pour constituer un accessoire qui peut être facilement appliqué à un appareil radiographique, mais bien entendu il peut aussi être incorporé dans un appareil radiographique comme partie de ses circuits.

Le dosimètre décrit est particulièrement approprié pour la radiographie endoorale, étant en condition de satisfaire aux exigences particulièrement sévères de cette application, mais il peut également trouver application dans tout autre domaine de la radiographie.

REVENDICATIONS

1 - Dosimètre pour emploi radiographique et spécialement mais non exclusivement pour la radiographie endoorale, comprenant un écran de conversion X-optique (3), destiné à être disposé derrière un film radiographique (P) du côté opposé à un générateur (4) de rayons X par rapport au sujet (D) à radiographier, des éléments photosensibles (2) disposés pour être excités par la lumière émise par ledit écran de conversion (3) lors qu'il est frappé par des rayons X, un circuit intégrateur (6) disposé pour intégrer le signal électrique produit par lesdits éléments photosensibles (2) et un circuit d'interruption (17-20) inséré dans le circuit d'alimentation (A-L) du générateur (4) de rayons X pour en interrompre le fonctionnement lors que le signal de sortie dudit circuit intégrateur (6) arrive à une valeur préfixée, caractérisé en ce qu'un support (29) pour le film radiographique (P), l'écran de conversion X-optique (3) et les éléments photosensibles (2) forment un organe capteur (25-29) de dimensions appropriées pour en permettre l'introduction dans la cavité orale; le circuit intégrateur (6) est constructivement séparé dudit organe capteur (25-29) et il est connecté auxdits éléments photosensibles (2) moyennant un câble (C); et le dispositif comprend des moyens circuitaux (6,13,16,17) pourvus de moyens (F,Q; 24,14) de réglage manuels et/ou automatiques pour adapter les caractéristiques du dosimètre aux différentes conditions opératives.

2 - Dosimètre pour emploi radiographique, suivant la revendication 1, caractérisé en ce que lesdits moyens circuitaux (6,13,16,17) du dosimètre comprennent un comparateur de seuil (13) ayant une tension de seuil (Vv, Vf) réglable, dans lequel le signal dudit circuit intégrateur (6) est confronté à ladite tension de seuil (Vv, Vf) pour produire un signal de sortie destiné à commander ledit circuit d'interruption (17-20).

3 - Dosimètre pour emploi radiographique, suivant la revendication 2, caractérisé en ce que ledit comparateur (13) comprend un générateur (24) de tension de référence (Vv) variable, en fonction de la tension d'alimentation (A) du générateur (4) de rayons X, de façon telle qu' elle tend à compenser les variations de la réponse globale du système aux variations de ladite tension d'alimentation (A).

4 - Dosimètre por emploi radiographique, suivant la revendication 3, caractérisé en ce que ledit générateur (24) de tension (Vv) de référence variable est disposé pour produire une tension de référence (Vv) qui varie linéairement avec la tension d'alimentation (A), en interpolant rectilinéairement la courbe (W ) de réponse complexive du système.

5 - Dosimètre pour emploi radiographique, suivant la revendication 4, caractérisé en ce que ledite tension (Vv) de référence variable linéairement est obtenue en amplifiant (en 33) avec un gain constant la différence entre une tension (Vr) proportionnelle à la tension d'alimentation (A) et una tension de référence fixe (Vf).

6 - Dosimètre pour emploi radiographique, suivant la revendication 3, caractérisé en ce que ledit générateur (24') de tension (Vv') de référence variable est disposé pour produire une tension de référence (Vv') qui varie non-linéairement avec la tension d'alimentation (A), en approximant la courbe (W) de réponse complexive du système.

7 - Dosimètre pour emploi radiographique, suivant la revendication 6, caractérisé en ce que ladite tension de référence (Vv') variable non-linéairement est obtenue à partir d'une tension (Vv) variable linéairement, en déterminant (en 35) son logarithme, en l'amplifiant (en 36) avec un gain constant et en déterminant ensuite (en 38) l'antilogarithme de la tension ainsi amplifiée.

8 - Dosimètre pour emploi radiographique, suivant la revendication 3, caractérisé en ce que ledit comparateur (13) inclut aussi un générateur (Y) de tension ($V_f$) de référence fixe et un commutateur (F") qui permet à l'opérateur de choisir pour la comparaison la tension de référence fixe ($V_f$) ou la tension variable (Vv), selon les circonstances.

9 - Dosimètre pour emploi radiographique, suivant la revendication 3, caractérisé en ce que ledit circuit intégrateur (6) comprend au moins deux condensateurs (10',10") qu'on peut commuter (par F') pour modifier la constante d'intégration du circuit (6).

10 - Dosimètre pour emploi radiographique, suivant les revendications 8 et 9, caractérisé en ce que ledit commutateur (F") des tensions de référence et ledit commutateur (F') des condensateurs (10',10") sont constitués par deux sections d'un même commutateur (F).

11 - Dosimètre pour emploi radiographique, suivant la revendication 3, caractérisé en ce que ledit circuit d'interruption (17-20) comprend des moyens (17) de retenue dans la condition d'interruption qui sont actifs pour un temps supérieur à la période maximum d'exposition permise par l'appareil radiographique (4) auquel le dosimètre est destiné.

12 - Dosimètre pour emploi radiographique, suivant la revendication 11, caractérisé en ce que lesdits moyens de retenue (17) du circuit d'interruption (17-20) sont prédisposés pour demeurer actifs pour une période non inférieure à 5 secondes.

13 - Dosimètre pour emploi radiographique, suivant la revendication 11, caractérisé en ce que ledit circuit intégrateur (6) comprend des moyens (11,12) de mise au zéro graduelle et spontanée qui agis-

sent dans un temps inferieur à la période d'activité desdits moyens de retenue (17) du circuit interrupteur (17-20).

14 - Dosimètre pour emploi radiographique, suivant la revendication 3, caractérisé en ce qu'il comprend un moyen interrupteur (41, 51) sensible à l'intensité de courant, connecté en série audit circuit d'interruption (17-20, K), et prédisposé pour interrompre le circuit lors du dépassement d'une valeur de courant comprise entre l'absorption de l'appareil radiographique (4) en conditions d'attente et son absorption en conditions d'activité; un relais (42, M) dont l'enroulement (42) est relié en parallèle au circuit d'alimentation (39) en aval dudit moyen interrupteur (41,51) sensible à l'intensité de courant et dont le contact (M) est disposé pour court-circuiter ledit moyen interrupteur (41,51) sensible à l'intensité de courant; et un moyen de retard (43,44) associé audit relais (42, M) et prédisposé pour en différer l'intervention pour un temps supérieur au temps d'intervention dudit moyen interrupteur (41,51) sensible à l'intensité de courant.

15 - Dosimètre pour emploi radiographique, suivant la revendication 14, caractérisé en ce que ledit moyen interrupteur (41) sensible à l'intensité de courant est constitué par un fusible.

16 - Dosimètre pour emploi radiographique, suivant la revendication 14, caractérisé en ce que ledit moyen interrupteur (51) sensible à l'intensité de courant est constitué par un interrupteur à relais de courant maximum.

17 - Dosimètre pour emploi radiographique, suivant la revendication 17, caractérisé en ce que ledit dispositif de retard (43,44) est constitué par un circuit comprenant une résistance (44) et une diode (45) connectées en série à l'enroulement (42) dudit relais, ed un condensateur (43) inséré en parallèle audit enroulement (42).

18 - Dosimètre pour emploi radiographique, suivant la revendication 14, caractérisé en ce qu'il comprend en outre un dispositif de signalisation optique et/ou acoustique (46), inséré en dérivation audit moyen interrupteur (41,51) sensible à l'intensité de courant pour en révéler la condition d'ouverture.

19 - Dosimètre pour emploi radiographique, suivant la revendication 11 et éventuellment 14, caractérisé en ce qu'il est réalisé sous la forme d'un accessoire séparé de l'appareil radiographique (4) et appliquable à un appareil radiographique, sans aucune modification de celui-ci, par simple branchement entre la fiche du câble (2) de l'appareil radiographique (4) et la prise d'alimentation (A).

20 - Dosimètre pour emploi radiographique, suivant la revendication 1, caractérisé en ce que ledit écran de conversion (3) et les éléments photosensibles (2) sont renfermés dans un organe capteur (28) étanche à la lumière et entièrement blindé (en 27) pour les inductions électromagnétiques, et que le câble (C) qui relie les éléments photosensibles (2) audit circuit intégrateur (6) est lui aussi blindé.

21 - Dosimètre pour emploi radiographique, suivant la revendication 20, caractérisé en ce que ledit organe capteur (25-29) comprend un circuit imprimé (1) qui porte les éléments photosensibles (2), une pièce d'espacement (25) en matériau tel que cuir synthétique, disposé pour couvrir ce circuit imprimé (2) et présentant des fenêtres en correspondance desdits éléments photosensibles (2), un écran de conversion (3) disposé sur ladite pièce d'espacement (25), un revêtement (27) de mince feuille d'aluminium qui enveloppe lesdits composants (1-3,25) en constituant un blindage électromagnétique, et un étui (28) en matière plastique qui renferme entièrement lesdites parties en se raccordant au guipage d'un câble blindé (c) relié audit circuit imprimé (1).

22 - Dosimètre pour emploi radiographique, suivant la revendication 21, caractérisé en ce que ledit étui (28) en matière plastique de l'organe capteur (25-29) présente sur sa face antérieure un moyen de retenue (29) pour un film radiographique (P) renfermé dans sa propre enveloppe étanche à la lumière.

23 - Dosimètre pour emploi radiographique, suivant la revendication 22, caractérisé en ce que ledit moyen de retenue (29) pour un film radiographique (P) est constitué par une poche (29), une bande, un guide ou une corniche formée par l'étui (28) en matière plastique de l'organe capteur.

FIG. 1

FIG. 8

FIG. 7

FIG. 2

FIG. 3

FIG. 4

FIG.5

FIG.6

FIG. 9

FIG.10